(19) Europäisches Patentamt
European Patent Office
Office européen des brevets

(11) EP 4 086 629 A1

(12) EUROPEAN PATENT APPLICATION
published in accordance with Art. 153(4) EPC

(43) Date of publication:
09.11.2022 Bulletin 2022/45

(51) International Patent Classification (IPC):
G01N 33/543 (2006.01)      G01N 33/553 (2006.01)
G01N 27/72 (2006.01)

(21) Application number: 19958597.7

(22) Date of filing: 31.12.2019

(52) Cooperative Patent Classification (CPC):
G01N 27/72; G01N 33/543; G01N 33/553

(86) International application number:
PCT/CL2019/050164

(87) International publication number:
WO 2021/134135 (08.07.2021 Gazette 2021/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicants:
• Universidad de Santiago de Chile
Santiago (ES)
• Universidad Central del Chile
Santiago (CL)
• Universidad Autonoma de Chile
Santiago (CL)

(72) Inventors:
• CASAGRANDE DENARDIN, Juliano
SANTIAGO (CL)
• ALTBIR DRULLNISKY, Dora Rosa
SANTIAGO (CL)
• BRIONES BARRALES, Jose Antonio
SANTIAGO (CL)
• MELO FREIRE, Rafael
SANTIAGO (CL)
• PALMA SOLORZA, Juan Luis
SANTIAGO (CL)
• MICHEA MORA, Sebastian Alfonso
SANTIAGO (CL)

(74) Representative: Pons
Glorieta Rubén Darío 4
28010 Madrid (ES)

(54) **SYSTEM AND METHOD FOR DETECTING A BIOLOGICAL ANALYTE, INCLUDING A MICROORGANISM, BY A CHANGE IN THE MAGNETIC PROPERTY OF A SUBSTRATE, USING SUPERPARAMAGNETIC NANOPARTICLES**

(57)    The invention relates to a system comprising superparamagnetic or anhysteretic nanoparticles (NPs) functionalised with an antibody, and a thin-film-type substrate of metal or an oxide thereof, functionalised with the same antibody; and to a method for detecting a biological analyte, such as a cell, protein, microorganism or similar, preferably a pathogenic microorganism, and even more preferably Listeria. The method comprises: (a) obtaining a control signal from a substrate (magnetic or not) coated with a thin film of metal or an oxide thereof, preferably gold, which can be functionalised with an antibody, the control signal being a magnetoresistance signal, a total magnetisation signal or a signal of the magnetisation curve; (b) mixing superparamagnetic or anhysteretic NPs functionalised with the antibody, with a liquid sample to analyse and confirm the presence or absence of the biological analyte, the NPs and the liquid sample making contact for 10-90 minutes; (c) dripping the dispersion obtained in step (b) onto the substrate of step (a), and then washing to remove NPs that are not chemically anchored to the surface of the biological analyte; (d) leaving the substrate to dry and re-measuring a signal in the same way as carried out in step (a); and (e) counteracting the control signal obtained in step (a) and the signal obtained in step (d), and in the absence of differences between the two measurements, confirming the absence of the biological analyte in the sample, the amount of microorganisms being directly proportional to the signal measured.

EP 4 086 629 A1

**FIGURE 1**

**Description**

## FIELD OF THE INVENTION

The invention is related to the field of preparations and systems that facilitate measurements and analysis tests for the detection of microorganisms, preferably pathogens, based on the magnetic properties of the materials that make up the detection system, such components being, in particular, superparamagnetic nanoparticles. or anhysteretic functionalized with an antibody specific for the pathogen to be detected, and a substrate also functionalized with the same antibody.

## BACKGROUND

To detect microorganisms in general, and pathogenic in particular, whether for humans, animals or plants, preferably technologies based on supporting standards techniques are used that confirm or rule out a first diagnosis based on the observation of symptoms or damage in humans, animals. or plants. Currently, to these standard techniques it is possible to add detection techniques based on the use of magnetic materials, and in particular, paramagnetic particles that are functionalized with specific binding moieties. This is commonly used in a technique that has been baptized as: magnetic separation technique, which would allow the isolation of an analyte from a liquid medium that contains it using specific molecular receptors or binding agents that immobilize it on magnetic carriers (particles), this using small amounts of magnetic carrier and analyte, and using a magnet for the separation of the analyte from the medium that contains it. These techniques have good results and acceptance due in general to high efficiency and feasibility and low cost. These systems have also been used for the detection of biological molecules or pathogens in solid phase assays using force (DR Baselt et al., Holidays. Sci. Technol., B 14, 789-794, 1996), optics (G U Lee et al., Bioanalytical Chemistry, 287, 261-271, 2000), and magnetic (DR Baselt et al., Biosensor Bioelectronics, 13, 731-739, 1998) properties of the materials.

But the magnetic carrier systems used in biomagnetic applications have limitations when it comes to detecting and identifying biological materials, such as a pathogen, this due - among other reasons, to the fact that the systems use particles larger than the size of most pathogen agents to achieve the magnetic separation effect, if the particles were smaller it would have a relatively low magnetic effect in terms of volume, and many times, it is not possible to determine if the magnetic particles are attached to the biological target providing incorrect measurements or false positives . The former has been solved using nano-sized magnetic carriers or nanoparticles, but there remains a need for reliable detection and identification of a specific biological target from a sample. The particles use a specific antibody that will only bind to the specific microorganism that you want to separate from the total solution. By applying a magnetic field to the entire solution, for example a permanent magnet, the group of specific microorganisms bound to the magnetic particles can be separated, while the rest of the solution is washed away. Thus, the final washed solution will be made up of the magnetic particles and the specific microorganism. This leads to DNA (deoxyribonucleic acid) multiplication assays known as PCR assays (Polymerase Chain Reaction assay).

US8679458B2 (Battelle Memorial Institute Inc) is referring to method and system to simply and efficiently determine the amounts of a preselected material in a particular solution by placing at least one organic material-functionalized superparamagnetic nanoparticle (Np) in a solution. of particular sample, where the preselected analytes are attached to the functionalization that corresponds to organic groups, then the superparamagnetic Nps are collected and the presence of a particular analyte is analyzed, with the preferred analyte being a heavy metal ion. Where a single particle does not serve for the magnetic detections that are intended to be measured in this presented protocol.

WO2003057175A3 (Visen Medical Inc) describes amine functionalized magnetic nanoparticle compositions and processes for synthesizing the same. The process consists of obtaining a carboxylated polymer in substantially pure form, which is used to prepare a substantially homogeneous carboxyl functionalized magnetic nanoparticle and polymeric coating. Carboxyl groups are converted to reactive primary amino groups through the use of a water soluble carbodiimide followed by the reaction of a large excess of a diamine. The amine-terminated nanoparticles are then reacted with bifunctional crosslinking agents and various biomolecules to produce nanoparticles for *in vitro* assays, cell sorting applications, and specific MR contrast agents.

US8815610B2 (International Business Machines Corp) is referring to magnetic nanoparticles that are detected through a thin membrane that separates them from a magnetic sensor. The technique can be used in a medical context, where an analyte of interest (present in a test fluid, such as blood) is bound to the membrane. In turn, other compounds are bound to the analyte, and one of these compounds includes a magnetic nanoparticle that is then detected by the sensor. In this way, the analyte is detected by detecting the magnetic nanoparticle. By counting the number of magnetic nanoparticles, the analyte concentration in the test fluid can be determined.

US8318093B2 (Leland Stanford Junior University) describes magnetic nanoparticles and methods for their use in the detection of biological molecules. Magnetic nanoparticles can bind to nucleic acid molecules, which are then captured by a complementary sequence attached to a detector, such as a Spin-pump detector or a Magnetic Tunnel Juntion (MTJ) detector. The detection of the bound magnetic nanoparticle can be achieved with high specificity and sensitivity. There are several magnetic methods to detect nanoparticles. Nanoparticles are anchored to nanometric elements (such as molecules) but in the present invention this occurs with three orders of magnitude greater and they are directly anchored to the pathogen, in this case listeria, in this case from 0.5 microns to 2 microns.

EP2385386A1 (General Hospital Corp) refers to a magnetic resonance based on sensor for detecting the presence or monitoring the concentration of an analyte in a sample solution. The sensor comprises magnetic nanoparticles attached to a moiety on which the analyte can reversibly bind, the nanoparticles being confined in a chamber with semipermeable walls that are impervious to the nanoparticles but permeable to the analyte. After binding of the analyte to the nanoparticles, the latter aggregate can be detected by a change in the relaxation time T2 of the sample solution within the chamber (magnetic relaxation switch). The analyte can be a carbohydrate such as glucose, an antibody, an amino acid, a nucleic acid, an oligonucleotide, a therapeutic agent, a peptide, a protein, etc. The assay can be read using MRS or MRI, e.g. ex. of well plates for high throughput analysis. The trial may be implantable.

Therefore, there remains a need for a system that allows reliable detection and identification of a specific biological target from a sample.

## BREIF SUMMARY OF THE INVENTION

A system of superparamagnetic or anhysteretic nanoparticles functionalized with an antibody and also a thin film-type substrate of metal or its oxide functionalized with the same antibody is described, and a method for the detection of microorganisms, preferably a pathogenic microorganism, is also described. more preferably Listeria. The method comprises: a) obtaining a control signal from a substrate (magnetic or not) coated with a thin film of a noble metal or oxide, including the example of gold, metal or oxide which can be functionalized with an antibody, where the control signal is a magnetoresistance signal, or of total magnetization or of the magnetization curve; b) Mix superparamagnetic or anhysteretic Nps, functionalized with an antibody, with a liquid sample that is required to be analyzed to confirm the presence or absence of a preferably pathogenic microorganism, allowing said Nps and the liquid sample to come into contact for a range of time comprised from 10 to 90 minutes; c) dripping the dispersion obtained in step "b)" onto the substrate measured in step "a)", and then proceed with a washing step that allows removing Nps that are not chemically anchored to the surface of the pathogenic microorganism; and finally in the last stage d) said substrate is allowed to dry and a signal is measured again from the substrate in the same way as was carried out in stage "a)"; the result will be obtained in a last stage e) that counters-subtracts the control signal obtained in stage "a)" and the signal obtained in stage "d)", if there are no differences between the two, the absence of microorganisms in the sample, and in addition, the amount of microorganisms in the sample will be directly proportional to the measured signal.

Said microorganism is preferably sought to be a bacterium. Such a microorganism can be grown in colonies in laboratories and selected from there. Said pathogenic microorganism can be selected from a food sample. And even more preferably, said food-borne pathogenic microorganism is selected from at least one of the group consisting of *Campylobacter jejuni, Clostridium botulinum, Costridium perfringens, Escherichia coli, Listeria monocytogenes, Norovirus, Salmonella enteritidis, Salmonella typhimurium, Shigella, Staphylococcus aureus, Vibro cholerae, Vibrio parahaemolyticus, Vibrio vulnificus, Yersinia enterocolitica,* among others. Being Listeria said pathogenic microorganism in food. The way to select said microorganism is by using a specific antibody for said microorganism, at the time of functionalization of the nanoparticle.

The present system comprises superparamagnetic or anhysteretic nanoparticles (NPs) which, upon coming into contact with the sample containing (or which are supposed to be present) the pathogenic microorganism to be analyzed, can mutually anchor to their surface. For this purpose, the sample must be liquid, so a solid sample must be diluted using an appropriate solvent. The sample when analyzed could contain a minimum volume of 0.1 mL and a maximum of 2.0 mL, and the detection protocol can identify the presence of the microorganism in vivo without the need for multiplication tests, through the presence of the NPs chemically attached to the surface of the pathogen. For the detection of such surface-functionalized NPs, various detection mechanisms can be used, including: detection by measurement of the magnetic moment of superparamagnetic NPs that are located on a substrate having on their surface a thin film coated preferably with gold, and they are detected in contrast to a control signal; or detection by using a substrate composed of multilayers with perpendicular anisotropy as a control signal, and that then, when adding the NPs, the resulting measurement will affect both its coercivity and its remanence, providing a completely different reading to the control signal.

The nanoparticles adhered to the microorganism, which in turn is adhered to the functionalized surface, can vary in the sample several magnetic effects detectable with a simple magnetic signal meter, such as magnetoresistance, change in coercivity, remanence, anisotropy, saturation magnetization.

The substrate can be selected from the materials used in the production process, whether of organic or inorganic origin. Said substrate will be a thin film coated with metal or its oxide that can be selected from the chemical interaction of said chemical elements with thiol groups, up to a thickness in the range of 1 - 1000 nm.

Said substrate can correspond to a non-magnetic or magnetic material composed of single or multilayer layers with perpendicular anisotropy as a control signal, and which then, by adding the NPs, allows a resulting measurement that affects both its coercivity and its remanence, providing a completely reading. different from control signal.

The present invention also relates to said superparamagnetic or anhysteretic NPs, with an average size in the range from 5 to 20 nm, functionalized on their surface with an antibody that confers specificity for the adhesion of a microorganism. Said antibody can preferably vary depending on the microorganism that is sought to adhere to the functionalized NPs. Preferably, said antibody allows surface binding with the Listeria bacteria, which causes a great problem in the food industry.

The present invention also refers to a method for the functionalization of superparamagnetic or anhysteretic NPs that allows antibodies to be anchored on a substrate having on its surface a thin film coated with metal or its oxide, which in turn is also functionalized on its surface with the same antibody as functionalized superparamagnetic or anhysteretic NPs. The microorganism can then adhere both to the surface of the NPs and to said functionalized gold-coated thin film. The method also comprises a washing step that is applied after adhering the NPs to the thin film and that allows to avoid the presence of NPs alone or that were not anchored to the surface of the thin film, thus avoiding false positives, in the detection. Said washing consists of a liquid solution (aqueous with controlled concentration of salts, and without solvents that affect the biological analyte (microorganism) or organic) that can be under a pre-established pressure and flow jet format that is injected onto the film by a pre-established time. The detection of the magnetic moment of the NPs chemically adhered to the surface can be carried out with standard measurements, including magnetic characterization systems using static techniques, such as VSM (vibrating sample magnetometer), AGFM (Alternate Gradient Force Magnetometer), MOKE (Magneto Optical Kerr Effect), SQUID (Superconducting Quantum Interference Device).

If the sample contains microorganisms, it is possible to detect said Nps by changing the control signal, since the presence of antibody-functionalized superparamagnetic or anhysteretic NPs are on the surface of the pathogenic microorganism and induce the change of the control signal. Such a change means the presence of the pathogenic microorganism in the analyzed liquid sample. However, if the control signal does not change, it means that there are no superparamagnetic or anhysteretic NPs on the surface of the thin film. In this situation, the washing process removes all the functionalized NPs from the surface of the film also functionalized with antibody, since the antibody-antibody interaction is electrostatic and therefore weak. In such a scenario, such NPs are weakly adhered to the surface of the thin film and, therefore, the washing process removes all the superparamagnetic or anhysteretic NPs functionalized with antibody. Without the presence of these NPs, the control signal does not change and, therefore, it can be understood that the analyzed sample does not contain the pathogenic microorganism.

## BREIF DESCRIPTION OF DRAWINGS

**Figure 1.** Methodology of the functionalization of the invention. (1) Superparamagnetic NPs that will be functionalized with the specific antibody (2) to obtain NPs functionalized with the antibody (3). In the same way, the substrate coated with a thin gold film (4) is functionalized with the antibody (2) to obtain a functionalized film with the same antibody anchored on the surface of the NPs (5). The functionalized NPs and the functionalized gold film do not stick together (6). However, for the NPs and the film to stick together, a specific bacteria must exist (7).

**Figure 2. Detection methodology by magnetic characterization.** The non-magnetic substrate (1) can be composed of a simple material (glass or silicon), where different materials such as CoCu-Au (2), Co-Au (3), Au (4) or CoPd-Au can be deposited. (5). Each of these systems is capable of providing a different detection signal, for which it will be possible to measure the magnetoresistance (6), total magnetization (7) or changes in anisotropy of the system (8, 9).

**Figures 3a and 3b. Transmission electron microscopy of NPs and Nps size scattering.** Figure 3a shows TEM image of a group of magnetite nanoparticles ($Fe_3O_4$). Figure 3b shows the Feret diameter frequency histogram, with an approximate mean of 13 nm.

**Figure 4. NPs magnetization curve.** Hysteresis curve measured for 15.5 mg of biologically functionalized nanoparticles.

**Figures 5a and 5b. Transmission microscopy of NPs adhered to listeria.** TEM images of a bacterium with functionalized $Fe_3O_4$ nanoparticles adhered to its surface measured for 15.5 mg of biologically functionalized nanoparticles.

**Figure 6. Methodology proof-of-concept magnetization curve.** Magnetization curves for a cobalt thin film (blue), cobalt thin film plus the listeria + Nps system (red) and the cobalt thin film plus the listeria + Nps system after a wash protocol (green).

**Figure 7. Detection methodology. Duty cycle for pathogen detection.** The functionalized surface is placed in the same solution and in contact with the bacteria solution and the nanoparticles in aqueous solution. A confidence interval of 90 minutes is expected for the bacteria to anchor to the functionalized surface and to the nanoparticles. The functionalized surface of the sample is washed to remove nanoparticles that have not been attached to a bacterium. The sample is measured, along with a control sample to see the magnetic signal change and thus detect the pathogen.

**Figure 8. Magnetoresistance curve.** MR curves measured at room temperature for a $[Co_2Cu_{1.5}] \times 30$ multilayer system under the influence of a magnetic field applied in the plane of the layers, as well as the current. The blue curve represents the curve obtained without $Fe_3O_4$ nanoparticles on the surface, while the pink one indicates the MR measurement obtained with nanoparticles on the surface of the multilayer system.

**Figures 9a to 9c.** Mechanisms of the detection methodology. Schematic design presenting the NPs detection mechanism. In the first instance only the magnetic film alone is measured (Figure 9a), in the second instance a quantity of magnetic NPs is deposited that change the magnetic properties of the film (Figure 9b). Finally, the number of particles is increased to saturate the magnetic signal that can be obtained (Figure 9c).

**Figure 10. Magnetization curves as a function of the amount of NPs added.** Magnetic hysteresis curves measured in an AGFM type magnetometer. The magnetic signal increases as NPs are added to the device. The signal from the film alone corresponds to the black color curve, as NPs are added on the film the signal increases.

**Figure 11. Fixed field magnetic signal.** Increase of the magnetic signal measured at 400 Oe as a function of the amount of mass of NPs added.

**Figure 12. Magnetization curves as a function of the amount of NPs added.** Magnetization curves measured in an AGFM type magnetometer. The magnetic signal changes as NPs are added on the multilayer system.

## DETAILED DESCRIPTION OF THE INVENTION

The present invention relates to a system and method for the detection of microorganisms, comprising superparamagnetic or anhysteretic nanoparticles functionalized with an antibody and a similarly functionalized substrate. The system allows the formation of conjugates of antibody-functionalized superparamagnetic or anhysteretic nanoparticles - microorganism - antibody-functionalized substrate, useful in a variety of applications for detection and measurement of samples.

## Definitions

A nanoparticle as described and claimed herein is a material that has all its dimensions in a range less than 100 nm, preferably between 1 and 100 nm, more preferably about 5 to 20 nm, about 10, most preferred. In a preferred embodiment, a superparamagnetic or anhysteretic iron oxide NP is used. Superparamagnetic or anhysteretic iron oxide is one of the highly magnetic forms that has a magnetic moment greater than about 30 emu / g Fe at 0.5 Tesla and about 300 K.

The "coating" is selected from the pathogenic microorganism to be detected. In this way, if it performs a surface engineering of the nanoparticle, where the specific antibody to said pathogen is chemically anchored, which confers specificity to the nanostructure. Such surface engineering It comprises the process of anchoring molecules that interact with the antibody and, therefore, this process aims to create active sites on the surface of the nanoparticle, where the antibody can anchor through a chemical bond. In one embodiment, the surface engineering process of a superparamagnetic or anhystematic nanoparticle, preferably of the general formula Fe3O4, included various commercially available reagents from Sigma-Aldrich and other companies, as described later herein. Such reagents include, but are not limited to, compounds synthetics ranging from iron (III) chloride (FeCl3, 97%, Aldrich) and iron (II) chloride (FeCl2, 98%, Aldrich) to the polyclonal antibody listeria, biotin (Thermo Fischer Scientific number PA1-85650). Additionally, it should be noted that such an antibody was chosen because the implementation was carried out taking listeria into consideration as a pathogenic microorganism. However, the surface engineering protocol described later in this document makes it possible to anchor different antibodies, depending on the pathogenic microorganism to be detected. In the event that salmonella were the pathogenic microorganism to be detected, the same protocol can be used to anchor the polyclonal antibody Samonela, also sold by the company Thermo Fischer Scientific, under reference PA1-73022. Similarly, the protocol can also be used in order to anchor different antibodies for the detection of Escherichia coli (polyclonal antibody E. Coli - Thermo Fisher Scientific number PA1-73035), Staphylococcus aureus (polyclonal antibody S. Aureus - Thermo Fisher Scientific number PA1-73174). Additionally, it is important to note that surface engineering involves the functionalization of the antibody through biotin, also known as vitamin B7, which is already chemically anchored to it. Therefore, the functionalization protocol described later in this document can be used for the anchoring process of different antibodies in order to detect a wide range of analytes, such as bacteria, cells, proteins, among others.

The present invention thus relates to a system of NPs, superparamagnetic or anhysteretic, functionalized with an antibody and a metal or oxide thin film substrate functionalized with the same antibody, and a method for the detection of microorganisms, preferably a pathogenic microorganism, even more preferably , Listeria, which comprises: a) obtaining a control signal from a substrate (magnetic or not) coated with a thin film of a noble metal, including gold or any other material that chemically interacts with thiol groups, such as, but not limited to, silver (Ag), copper (Cu) or zinc oxide (ZnO), functionalized with an antibody, where the control signal is a magnetoresistance signal, total magnetization or anisotropy change; b) mixing superparamagnetic or anhysteretic NPs functionalized with an antibody with the liquid sample that is required to be analyzed to confirm the presence or absence of a pathogenic microorganism to be detected, allowing said NPs and the liquid sample to come into contact for a range of time that it goes from 10 to 90 minutes; c) dripping the dispersion obtained in step "b)" onto the substrate measured in step "a)", to then proceed with a washing step that allows removing NPs that are not chemically anchored to the surface of the pathogenic microorganism ; and d) said substrate is allowed to dry and a signal is re-measured from the substrate in the same way as was done in step "a)"; and e) contrast the control signal obtained in stage "a)" and the signal obtained in stage "d)", if there are no differences between the two, the absence of microorganisms in the analyzed sample is confirmed.

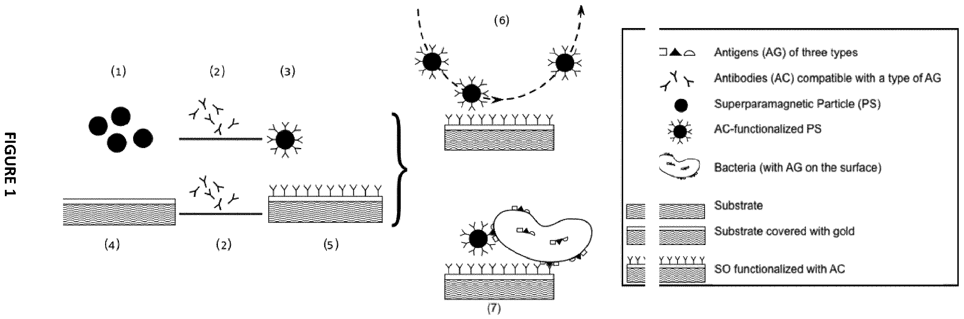

Furthermore, the detection method comprises a detection of said NPs + microorganism by modifying the magnetic properties. To ensure the absence of false positives, the substrate, whether it is a thin film coated with gold, but not limited to such metal, or a substrate composed of multilayers, is subjected to a washing step in order to remove all the NPs that are not present. chemically anchored on the surface of the microorganism since the only strong point of interaction is the binding of the antibody with the surface of the microorganism and if the mixture does not contain said microorganism, the NPs interact weakly with the substrate and a flow of washing liquid would be sufficient to remove said NPs. The washing solution or flow of washing liquid can be selected from its composition, since it must not contain any solvent that harms the pathogenic microorganism, preferably an aqueous solution with a concentration of salts and controlled pH values. On the other hand, if said microorganism is anchored to said substrate, the NPs + microorganism + substrate interaction is stronger due to the presence of the antibody on both surfaces of the particle and of the thin film that are chemically anchored to the surface of the microorganism and the washing stage fails to remove said microorganism. In this case, the measured magnetic signal will show changes compared to the control signal, also magnetic, since said microorganism contains NPs, superparamagnetic or anhysteretic, on its surface, modifying the magnetic response. Figure 1 shows the steps necessary for a positive detection, that is, a sample with the presence of microorganisms. From numbers 1 to 5 the surface engineering is outlined to anchor the antibody on the surface of the NPs, as well as on the thin film coated with gold, or any other metal that chemically interacts with thiol groups, on the substrate. Number 6 of Figure 1 shows that the interaction between NPs and gold-coated thin film, or any other metal that chemically interacts with thiol groups, both functionalized with antibodies, is weak. Then the NPs do not stick to the surface of the substrate. However, in the presence of the microorganism, said microorganism interacts with the surface of said Nps causing it to remain adhered even after the washing stage has been carried out.

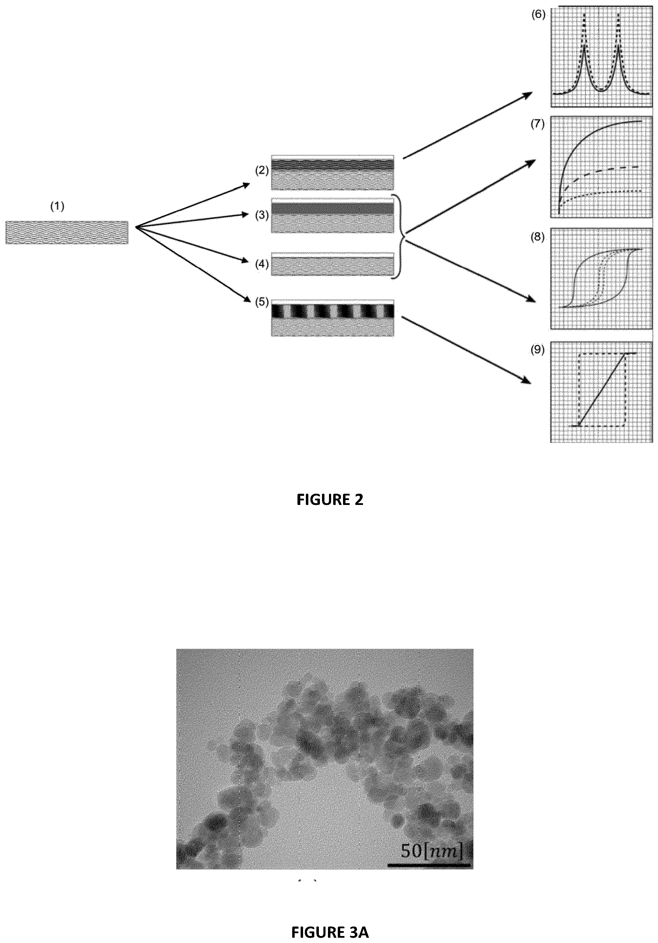

The detection method described above involves the use of a non-magnetic substrate and, therefore, the magnetic signal comes only from the NPs. However, a magnetic thin film can also be occupied. In such a scenario, the signal that is measured will depend on the magnetic configuration of said NPs or thin film depending on the magnetic configuration of the system, and then it is possible to measure the variation of signals of the group consisting of: magnetoresistance, coercive field, remanent magnetization and total magnetization to confirm the presence or absence of the pathogenic microorganism to be detected, provided that such signal is contrasted with a control signal. The different magnetic configurations together with the different observable effects are shown in figure 2.

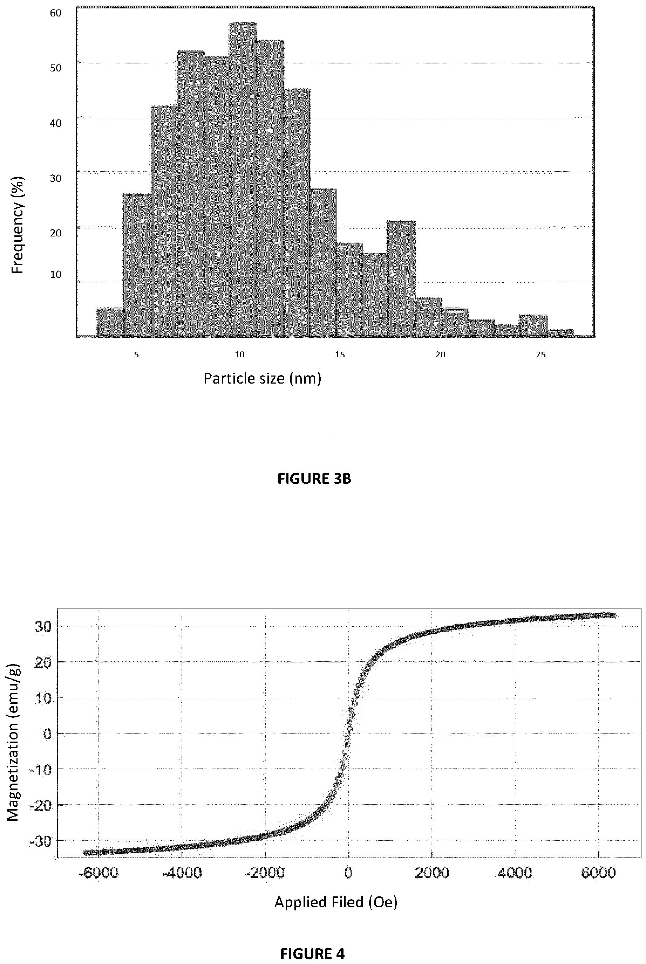

Figures 3a and 3b show an image of the NPs of the general formula $Fe_3O_4$ acquired by means of a transmission electron microscope, the size of these nanostructures was obtained through the measurement of the Ferret diameter, where the average size found was 13 nm.

Then, the NPs go through a process of functionalization with the antibody. For the nonlimiting example of the invention, said antibody corresponds to a molecule that has high specificity for a bacterium called Listeria. Thus a polyclonal antibody specific for Listeria (Thermo Fischer Scientific) was used. Figure 4 presents the magnetic hysteresis curve at room temperature for the functionalized NPs, where clearly, the superparamagnetic behavior of the system can be noted, since the magnetic cycle does not present a difference between the roundtrip paths. Furthermore, even after functionalization, the saturation magnetization found was approximately 33 emu/g, which implies that this process does not vary the magnetic properties of the nanostructure.

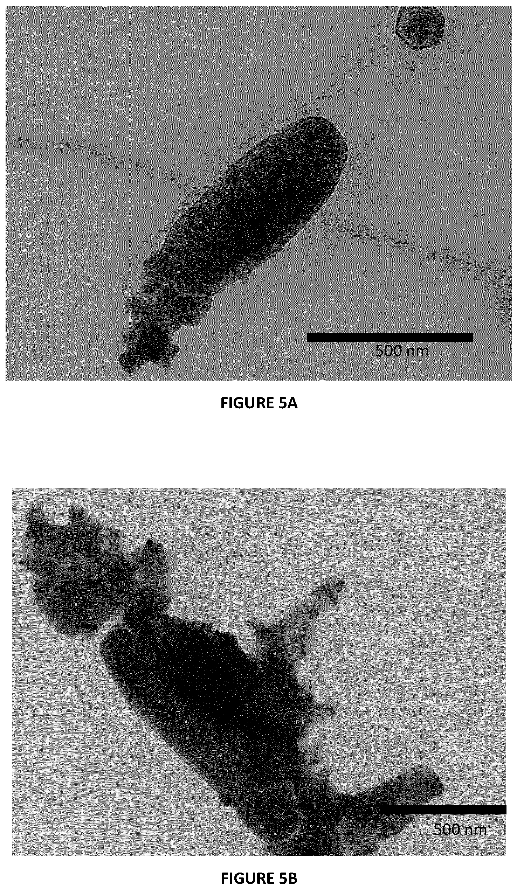

For the detection of NPs in conjunction with the microorganism, said functionalized NPs were interacted with different solutions containing different concentrations in a range of $10^2$-$10^9$ CFU of listeria for an incubation period that can vary from 10 to 90 minutes. To verify that the system has effectively stuck, the exploration was carried out via electron transmission microscopy to the incubated or resulting solution after 90 minutes of an incubation period, via magnetic separation, here it is possible to observe the existence of NPs in abundance on the surface of bacteria in the two images presented in Figures 5a and 5b. In addition, it can be noted that there are no isolated NPs in the grid used to obtain the micrographs, that is, the only NPs observed are those that are on the surface of the bacteria. This shows that the functionalization and incubation process is effective.

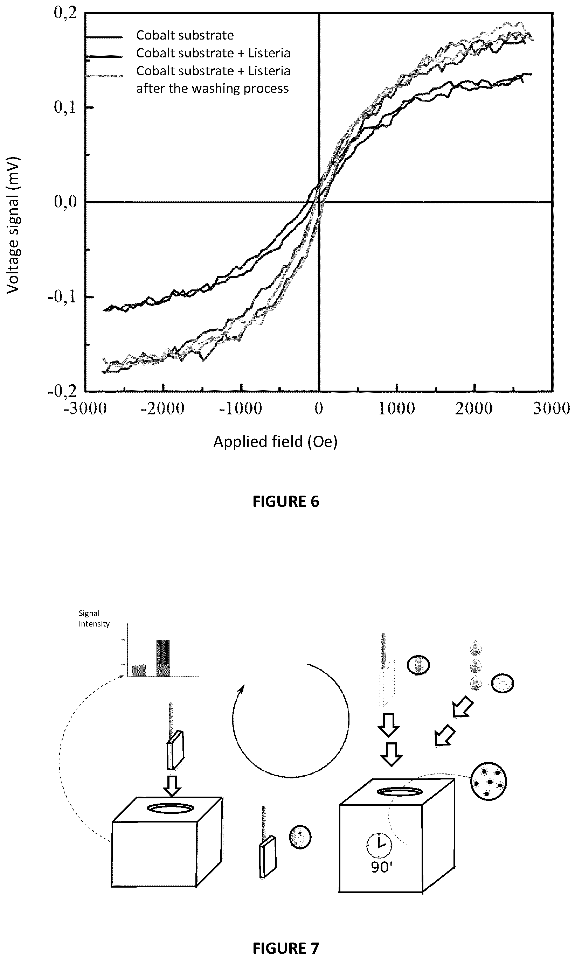

Subsequently, the same solution used to take the TEM micrographs was deposited on a substrate (cobalt film) covered with a gold film functionalized with the antibody. It was waited 20 min, allowing the interaction of the bacteria with the film, and the film was washed with a phosphate buffered solution with a fixed pH value of 7.4. Figure 6 presents the magnetization curve obtained for the system described above under 3 different configurations. The control signal corresponding to the thin film alone appears in blue. The red curve represents the control system coated with 10 $\mu$l of the bacteria + functionalized NPs solution. And finally, the green curve shows the same system as the previous one after the washing stage. Clearly, the magnetic signal obtained is larger compared to the control signal, thus evidencing the presence of listeria in the substrate. To confirm the strong interaction between the film and the bacteria, the washing step was carried out without obtaining differences in the magnetic signal. Therefore, this shows that the strong interaction between the film and the bacteria, in addition to providing proof that the jet of solution used during the washing stage is not strong enough to remove the batteries from the film producing a false negative.

In general, Figure 7 shows the operating cycle for the detection of a pathogenic microorganism, such as listeria.

In summary, during the incubation period, the substrate (magnetic or not) coated with a thin film of gold functionalized with the antibody must be introduced into the detector system to obtain the control signal, where said detector system can consider magnetoresistance measurements. , total magnetization or change in anisotropy of the system. Then, the superparamagnetic Nps are mixed with a solution containing the sample that is required to be analyzed and where the presence of a microorganism that could be detected could be confirmed and said Nps and said solution are allowed to interact for up to 90 minutes. After the incubation period, the dispersion resulting from the mixture described above must be dripped onto the previously measured substrate, proceeding to the washing stage in order to remove NPs that are not "stuck" to the surface of the pathogen. After allowing to dry, the substrate is reintroduced into the detector system to obtain a signal measurement that can be selected from magnetoresistance, total magnetization, or anisotropy change of the system. And finally, the signal obtained is contrasted with the control signal to confirm the presence or absence of the microorganism.

**Example 1: Synthesis and functionalization of Fe$_3$O$_4$ nanoparticles (NPs) with** polyclonal listeria **antibody.**

**Materials.** Iron (III) chloride (FeCl$_3$, 97%, Aldrich), iron (II) chloride (FeCl$_2$, 98%, Aldrich), ammonium hydroxide (NH$_4$OH, 30%, Aldrich), ethanol (C$_2$H$_6$O, 99.9% , JT Baker) and APTES (C$_6$H$_{14}$, 98.7%, BioslabChile), succinic anhydride (C$_4$H$_4$O$_3$, 99%, Aldrich), anhydrous tetrahydrofuran (C$_4$H$_8$O, 99.9%, Aldrich), N-ethyl-N'-hydrochloride (3-dimethylaminopropyl) carbodiimide (EDC, CaH$_{17}$N$_3$HCl, commercial grade, Aldrich), N-Hydroxysuccinimide (NHS, C$_4$H$_5$NO$_3$, 98%, Aldrich), 3-mercaptopropionic acid (C$_3$H$_6$O$_2$S, 99%, Aldrich), bovine serum albumin (BSA, 96%, Aldrich), streptavidin (Thermo Fischer Scientific number 434302), listeria polyclonal antibody, biotin (Thermo Fischer Scientific number PA1-85650), phosphate buffered saline (PBSx10, pH 7.4, Thermo Fischer Scientific), azide sodium (NaN$_3$, 99.5%, Aldrich). All reagents are used as received.

**Synthesis of NPs functionalized** by a **carboxyl group Fe$_3$O$_4$** [X. Qu, F. Wang, Y. Sun, Y. Tian, R. Chen, X. Ma, C. Liu, Selective extraction of bioactive glycoprotein in neutral environment through Concanavalin A mediated template immobilization and dopamine surface imprinting, RSC Adv. 6 (2016) 86455-86463. doi: 10.1039/C6RA11040A], First, two aqueous solutions of FeCl$_3$.6H$_2$O and FeCl$_2$ of 2: 1 molar concentration are prepared and distilled in water. The solutions are then mixed in a 1: 2 (molar) ratio of Fe$^{2+}$ and Fe$^{3+}$, respectively. After that, the mixture is heated to 80 ° C and, under vigorous stirring, with a quantity of 30% by weight NH$_4$OH in solution, which adheres until obtaining a pH value of 10. The formation of a precipitate of black color. The reaction is carried out for 60 minutes. After these 560 minutes, the heat source is turned off, the solution is cooled and the black precipitate is separated and washed using ethanol and distilled water. Once the Fe$_3$O$_4$ NPs are purified, they are dispersed in 200 mL of an ethanol/H$_2$O mixture (1: 1) at room temperature. The pH of the dispersion is adjusted with ethanoic acid to reach a value of 4.0. Then, it is protected in an atmosphere of N$_2$, 4 mL of APTES are added to the reaction that takes all night. Subsequently, the amino-modified Fe$_3$O$_4$ NPs are washed with distilled water and ethanol and dried in vacuo. After that, 300 mg of the NH$_2$-functionalized NPs are dispersed in a tetrahydrofuran (THF) solution, dropwise, then 60 mL of another THF solution containing succinic anhydride is added. The System is allowed to react for 24 h and, once again, the NPs are separated, washed and dried under vacuum, to obtain functionalized NPs-Fe$_3$O$_4$ NPs with a carboxylic group.

**Preparation of magnetic NPs with anti-listeria** (Y. Mao, X. Huang, S. Xiong, H. Xu, ZP Aguilar, Y. Xiong, Large-volume immunomagnetic separation combined with multiplex PCR assay for simultaneous detection of Listeria monocytogenes and Listeria ivanovii in lettuce, Food Control. 59 (2016) 601-608. doi:https://doi.org/10.1016/j.foodcont.2015.06.048). Initially, 30 mg of carboxylated $Fe_3O_4$ NPs are dispersed in 30 mL of PBS (0.01 M, pH 7.4). Then, the PBS solution containing 8.7 mg of EDC.HCl and 9.75 mg of NHS, slowly adheres to the solution. The reaction is carried out for one hour and the NPs are separated and washed with PBS to remove excess chemical regents. Subsequently, 2.4 mg of streptavidin are adhered within the dispersion containing $Fe_3O_4$ NPs with activated-carboxyl modified. The mixture is allowed to react at room temperature for 2 h, and a 1% (w / v) solution of BSA is added and the reaction is carried out for 45 minutes. NPs functionalized with streptadivine are separated and washed using PBS then resuspended in 0.01 M sterilized PBS. Once again, the surface-modified NPs are dispersed in PBS and 3 mg of antibody is added. The mixture is incubated at room temperature for 30 min under gentle mechanical shaking. Finally, the antibody-functionalized NPs are separated, washed with sterile PBS, and the resulting product is re-suspended in sterile PBS containing 0.2 ‰ $NaN_3$, then stored at 4 ° C for use.

Modification of the magnetic film surface (Y. Xue, X. Li, H. Li, W. Zhang, Quantifying thiol - gold interactions towards the efficient strength control, Nat. Commun. 5 (2014) 4348. http://dx.doi.org/10.1038/ncomms5348). A carboxylic group functionalized gold surface is prepared by immersing the substrate in an acidic solution composed of 100 mM 3-mercaptopropionic acid prepared in PBS (7,4). The reaction is carried out for 5 hours. Then, the substrate is carefully washed with ethanol and distilled water and dried to later modify it to put the polyclonal listeria antibody. To achieve this goal, the same method described in the previous section is used.

## Example 2: Solvothermal synthesis of $CoFe_2O_4$'s NPs

**Materials.** Iron (III) acetylacetonate ($Fe(C_5H_7O_2)$ 3,97%, Aldrich, $Fe(acac)_3$), cobalt (II) acetylacetonate ($CO(C_6H_7O_2)_2$, 97%, Aldrich, $Co(acac)_2$), toluene ($C_6H_5CH_3$, 99.9%, JT Baker), oleic acid (C18H34O2, 90%, Aldrich, OAc), oleylamine (C18H37N, 70%, Aldrich, OAm), ethanol (C2H6O, 99.9%, JT Baker) and hexane (C6H14, 98.7%, BioslabChile). All reagents are used as received

**Synthesis.** The $CoFe_2O_4$ NPs are prepared using a stainless steel reaction kettle with a Teflon coating (20 ml) according to the procedure described by Peng et al. (M. Jiang, X. Peng, Anisotropic Fe3O4/Mn3O4 Hybrid Nanocrystals with Unique Magnetic Properties, Nano Lett. 17 (2017) 3570-3575. Doi:10.1021/acs.nanolett.7b00727). Quickly, 0.5 mmol of $Fe(acac)_3$ and 0.25 mmol of $Co(acac)_2$ are dissolved in toluene, then, it is mixed under vigorous stirring. Subsequently, 0.5 mL and 3.0 mL of OAc and OAm are added respectively. The mixture is stirred magnetically for 20 minutes at room temperature, where a red solution is obtained within the Teflon coating. The reactor is put into a muffle and heated to 200°C. The solovothermal reaction is carried out for 20 h. The sample is allowed to cool to room temperature and the $CoFe_2O_4$ NPs are rinsed many times with ethanol and hexane. Finally, the NPs are dispersed in hexane and stored at 4 ° C for future use.

## Example 3: Synthesis of magnetic and non-magnetic films.

Magnetic and non-magnetic film is grown by high vacuum cathodic evaporation. This process is known as sputtering. The three types of magnetic film and the non-magnetic film provide 4 types of measurable signals. The average parameters used in each growth process are: base vacuum of approx. 9 KPa (6.7 x10-7 Torr), argon pressure approx. 0.4 KPa (3 mTorr), 20 sccm argon flow, average power 50 W (20-100W). The whites used to evaporate come from the Kurt J. Lesker company (cobalt, palladium, manganese oxide, tantalum, gold, copper) where each one has at least two digits of purity (99.99%).

Synthesis:

a) Gold coating: All substrates used have a 30 nm gold coating performed under standard conditions of the 108 Auto Sputter Coater Cressington equipment.

b) Cobalt magnetic films: 20 nm was deposited on a 5x5 mm glass and silicon substrate under base vacuum conditions of approx. 9 KPa (6.7 $\times 10^{-7}$ Torr), argon pressure 0.4 KPa (3 mTorr), argon flow 20 sccm. The power used for such a deposit was 20 W generating a rate of 0.72 nm/min. The sample was then coated with 2 nm manganese oxide to prevent oxidation (150 W (RF) at a rate of 0.8 nm / min). Finally, the sample was coated with 30 nm of gold as indicated in subsection "a)" of this section.

c) Copper and cobalt multilayer magnetic films: On a 5x5 mm glass and silicon substrate, 2 nm of copper and 1.5 nm of cobalt were alternately deposited until obtaining 30 bilayers of both materials under conditions of base vacuum of approx. 9 KPa (6.7 $\times 10^{-7}$ Torr), argon pressure approx. 0.4 KPa (3 mTorr), 20 sccm argon flow. The powers used for copper and cobalt were 50 W at a rate of 3 nm/min and 20 W at a rate of 0.72 nm/min respectively. The sample was then coated with 1.5 palladium to prevent oxidation (45 W at a rate of 2 nm/min). Finally, the sample was coated with 30 nm of gold as indicated in subsection "a)" of this section.

d) Magnetic films with perpendicular anisotropy: 1.5 nm of palladium was deposited on a 5x5 mm glass and silicon substrate at a power of 45 W with a rate of 2 nm/min, then alternately 1.5 is deposited palladium nm and cobalt 0.3 nm in a total of 10 repetitions, the cobalt power was 20 W with a rate of 0.72 nm/min. The sample was then coated with 1.5 palladium to prevent oxidation (45 W at a rate of 2 nm/min). Finally, the sample was coated with 30 nm of gold as indicated in subsection "a)" of this section.

**Example 4: Magnetoresistance measurements in system $[Co_2Cu_{1,05}]_{x30}$.**

The multilayer system of cobalt with copper is composed of a 2 nm cobalt layer, and on top of this a 1.5 nm copper layer is synthesized, this bilayer is repeated 30 times $[Co_{2.0}Cu_{1.5}]$ x30. With this a total thickness of 105 nm is obtained. The synthesis of the multilayers was carried out by sputtering a silicon and/or glass substrate. First, 2 nanometers of copper were deposited and then 1.5 nanometers of cobalt were deposited on it. The conductive material is used to isolate the magnetic interactions of each cobalt layer, allowing the modification of the magnetization to be highly dominated by the external applied field and not by the magnetic field generated by each adjacent layer.

This system presents the magnetoresistance effect (numeral 6 of figure 2), which is a property of magnetic materials and is manifested as the change in resistance due to the interaction with an external magnetic field applied in a certain direction. It is measured as a percentage change, based on:

$$MR\ (\%) = 100 \times (R_H-R_0)/R_0 \qquad\qquad (1)$$

where RH corresponds to the electrical resistance of the material when it interacts with the external magnetic field and R0 is the resistance without field. There are different phenomena associated with magnetoresistance, including: ordinary magnetoresistance (MRO), anisotropic magnetoresistance (MRA), giant magnetoresistance (MRG) and colossal magnetoresistance (MRC).

For resistivity measurements, the Van der Pauw or four-point method was used (van der Pauw, LJ (1958), Philips Research Reports. 13: 1-9, van der Pauw, LJ (1958), Philips Technical Review 20: 220-224). This allows measurements to be made with the only restriction being that the layers resemble as much as possible a two-dimensional system, that is, a very small thickness compared to their cross section. The substrate is installed on a rod specially designed to make this measurement and is positioned in a transport measurement system based on the magnetic field. The measurement range varies from -6000 [Oe] to 6000 [Oe].

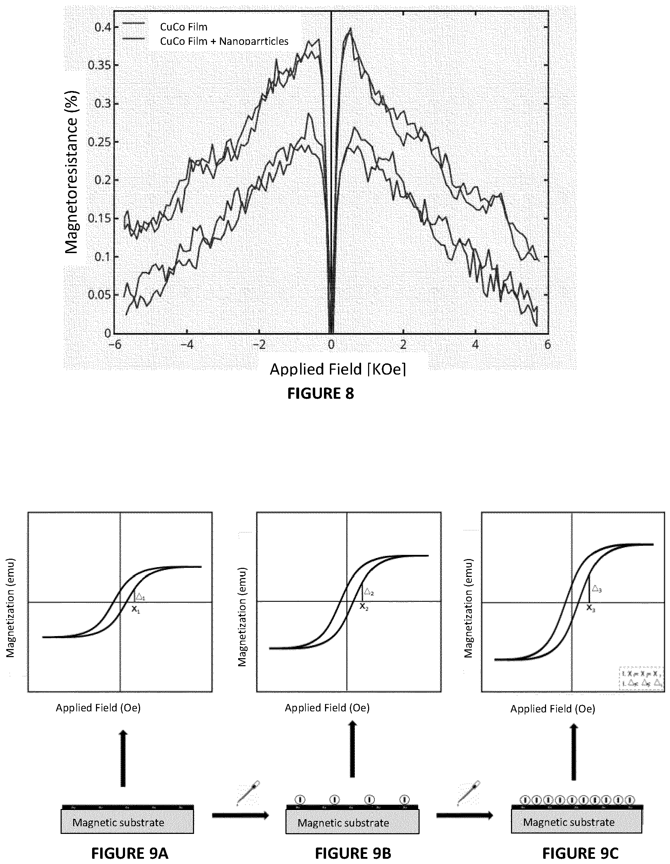

As shown in figure 8, due to the inclusion of the nanoparticles, a change in the MR signal of the multilayers is observed. Said maximum percentage change achieved by the sample is approximately 0.25% above the resistance of the material to zero field.

**Example 5: Measurements of signal change in magnetic films due to the presence of nanoparticles.**

If the signal of the magnetic film is measured by itself, in this case a cobalt film 10 nm thick and covered by manganese oxide (Co/MgO). The type of signal that is detected in this case corresponds to that shown in numeral 6 of figure 2. The detection mechanism is shown in figure 9. For all other cases the detection mechanism is the same. First the properties of the magnetic film are measured, only that signal is considered the initial of the measurement cycle. Subsequently, magnetic nanoparticles are added on the surface of the multilayer system and the measurement is made again under the same field and frequency conditions used to obtain the initial signal. Therefore, a new magnetic signal for the film-nanoparticle system is recorded and compared with the initial signal in order to study changes in the magnetic properties of the multilayer system induced by the nanostructure deposited on its surface. The magnetic characterization was carried out in an alternating force gradient magnetometer with a capacity of 1 T and a sensitivity of $1 \times 10^{-6}$ emu

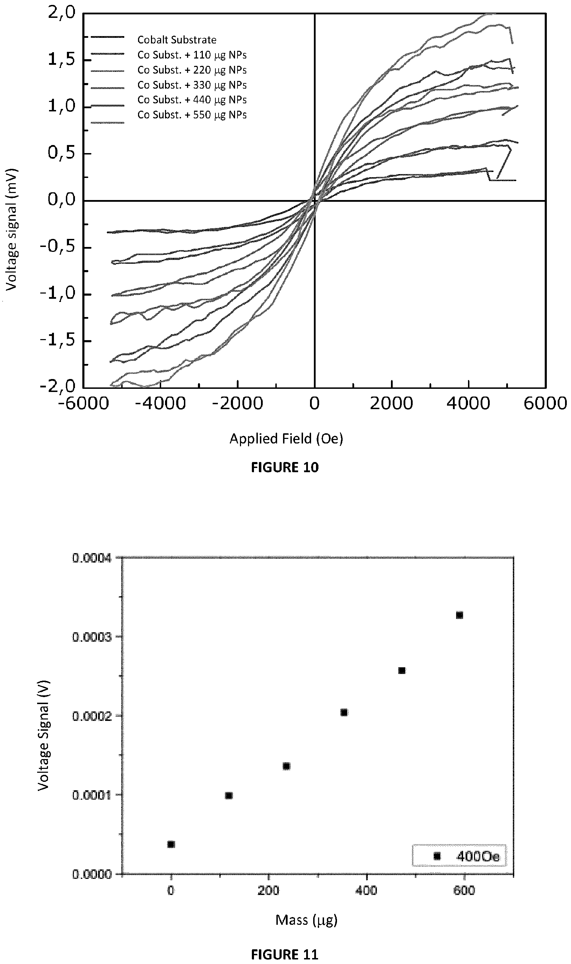

Using the methodology for comparing the magnetic signal obtained before and after the deposit of nanostructures on the surface of the multilayer system, this signal was measured under different amounts of $CoFe_2O_4$ and the recorded curves are shown in Figure 10. It can be noted that the Magnetic signal increases with the amount of nanoparticles deposited on the surface of the multilayer system. More specifically, it is possible to observe that the saturation magnetization of the measured system increases regularly with the mass of nanostructure deposited on the surface of the multilayer system.

Given the behavior observed in Figure 10, the magnetic signal was taken at a fixed field of 400 Oe and a graph of magnetization versus deposited mass was made. Such a graph is presented in Figure 11. As can be seen, the magnetization increases linearly with the mass of material deposited on the multilayer system.

**Example 6: Change in anisotropy due to the presence of NPs.**

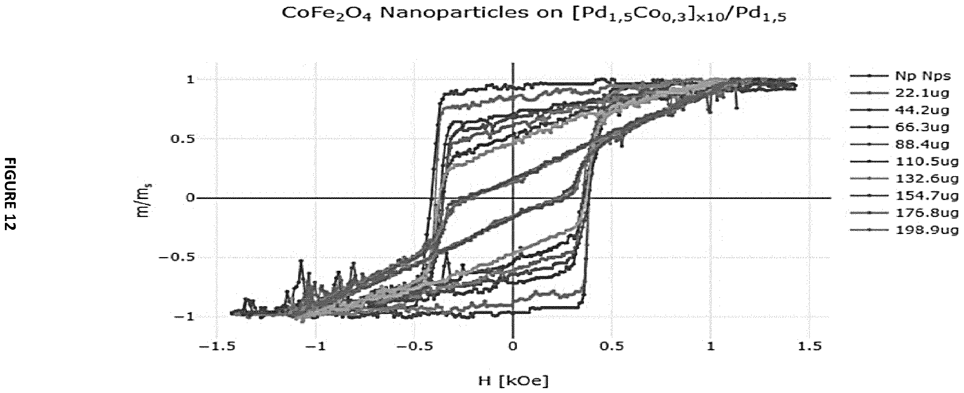

In a Co/Pd multilayer system, under a certain multilayer geometry, an axis of easy magnetization is found in the direction perpendicular to the plane of the film. Due to the presence of NPs on the magnetic film, this anisotropy changes from being on the axis perpendicular to the plane, to being in the plane of the film. The changes induced by the NPs on the film are clearly observed in Figure 12 and were made as a function of the amount of NPs added to the system.

**Claims**

1. Superparamagnetic or anhysteretic nanoparticle system for the detection of a biological agent including cells, proteins or microorganisms, preferably microorganisms, and even more preferably pathogenic microorganisms comprising superparamagnetic or anhysteretic nanoparticles (NPs) functionalized with an antibody for said biological analyte and also a thin film type substrate metal or its oxide, functionalized with the same antibody.

2. The system of claim 2 wherein said microorganism is selected from a food pathogenic microorganism.

3. The system of claim 2 wherein said food-borne microorganism is selected from at least one of the group consisting of *Campylobacter jejuni, Clostridium botulinum, Costridium perfringens, Escherichia coli, Listeria monocytogenes, Norovirus, Salmonella enteritidis, Salmonella typhimurium, Shigella, Staphylococcus aureus, Vibro cholerae, Vibrio parahaemolyticus, Vibrio vulnificus, Yersinia enterocolitica.*

4. The system of claim 3 wherein said food pathogenic microorganism is selected from Listeria.

5. The system of claim 1 wherein said substrate is selected from a non-magnetic or magnetic material composed of single or multilayer layers with perpendicular anisotropy.

6. The system of claim 6 wherein said substrate has on its surface a thin film coated with metal or its oxide which in turn is also functionalized on its surface with the same antibody as functionalized superparamagnetic or anhysteretic NPs.

7. The system of claim 1 wherein said superparamagnetic or anhysteretic NPs have a size less than 10 nm.

8. The system of claim 7 wherein said superparamagnetic or anhysteretic NPs have an average size between 1 to 100 nm.

9. The system of claim 8 wherein said superparamagnetic or anhysteretic NPs have an average size between 5 to 20 nm.

10. The system of claim 9 wherein said superparamagnetic or anhysteretic NPs have an average size of 10 nm.

11. The system of claim 1 wherein said superparamagnetic or anhysteretic NPs are selected from superparamagnetic or anhysteretic iron oxide NPs.

12. The system of claim 1 wherein said antibody is selected from the polyclonal antibody Listeria (Thermo Fischer Scientific number PA1-85650); Samonela polyclonal antibody (Thermo Fischer Scientific, under reference PA1-73022; E. Coli polyclonal antibody (Thermo Fisher Scientific number PA1-73035) or Staphylococcus aureus polyclonal antibody (Thermo Fisher Scientific number PA1-73174).

13. The system of claim 1 wherein said coated substrate is selected from a magnetic substrate.

14. The system of claim 1 wherein said coated substrate is selected from a non-magnetic substrate.

15. The system of claim 1 wherein said coated substrate is coated with a thin film of a noble metal or a material that chemically interacts with thiol group.

16. The system of claim 15 wherein said noble metal is selected from gold.

17. The system of claim 16 wherein said material that chemically interacts with thiol groups is selected from silver (Ag), copper (Cu) or zinc oxide (ZnO).

18. Method for the detection of a biological analyte, including cell, protein, microorganisms, preferably, a pathogenic microorganism, and even more preferably, *Listeria,* comprising:

   a) obtaining a control signal from a selected glass or silicon substrate, coated with a thin film of a metal or its oxide, which can be functionalized with an antibody, where the control signal is a magnetoresistance signal, of total magnetization or the magnetization curve;
   b) mixing superparamagnetic or anhysteretic NPs, functionalized with an antibody, with a liquid sample that needs to be analyzed to confirm the presence or absence of said biological analyte, allowing said NPs and the liquid sample to come into contact for a time range from 10 to 90 minutes;
   c) dripping the dispersion obtained in step b) onto the substrate measured in step a), to then proceed with a washing step that allows removing NPs that are not chemically anchored to the surface of said biological analyte; and
   d) drying said substrate and re-measure a signal from the substrate in the same way as was done in step a);
   e) to counteract the control signal obtained in stage a) and the signal obtained in step d), confirming the absence of said biological analyte in the sample, if there are no differences between the two.

19. The method of claim 18 wherein said sample is selected from a liquid sample.

20. The method of claim 18 wherein said detection of functionalized NPs is performed by detection by measurement of the magnetic moment of superparamagnetic NPs that are located on a substrate and are detected by contrast with a control signal.

21. The method of claim 18 wherein said detection of functionalized NPs is carried out by means of detection by using a substrate composed of multilayers with perpendicular anisotropy as a control signal.

22. The method of claim 18 wherein said washing consists of washing with a liquid solution, preferably an aqueous solution with a controlled concentration of salts, and without solvents that affect the biological analyte.

23. The method of claim 22 wherein said liquid washing solution is selected from a jet of liquid solution of preset pressure and flow that is injected onto the film for a preset time.

24. The system of claim 18 wherein said biological analyte is selected from a food pathogenic microorganism.

25. The system of claim 24 wherein said food-borne microorganism is selected from at least one of the group consisting of *Campylobacter jejuni, Clostridium botulinum, Costridium perfringens, Escherichia coli, Listeria monocytogenes, Norovirus, Salmonella enteritidis, Salmonella typhimurium, Shigella, Staphylococcus aureus, Vibro cholerae, Vibrio parahaemolyticus, Vibrio vulnificus, Yersinia enterocolitica.*

26. The system of claim 24 wherein said food-borne microorganism is selected from *Listeria.*

27. The system of claim 18 wherein said substrate is selected from a non-magnetic or magnetic material composed of single or multilayer layers with perpendicular anisotropy.

28. The system of claim 27 wherein said substrate has on its surface a thin film coated with metal or its oxide which in turn is also functionalized on its surface with the same antibody as functionalized superparamagnetic or anhysteretic NPs.

29. The system of claim 18 wherein said superparamagnetic or anhysteretic NPs are less than 10 nm in size.

30. The system of claim 29 wherein said superparamagnetic or anhysteretic NPs have an average size between 1 to 100 nm.

31. The system of claim 30 wherein said superparamagnetic or anhysteretic NPs have an average size between 5 to 20 nm.

**32.** The system of claim 31 wherein said superparamagnetic or anhysteretic NPs have an average size of 10 nm.

**33.** The system of claim 18 wherein said superparamagnetic or anhysteretic NPs are selected from iron oxide superparamagnetic or anhysteretic NPs.

**34.** The system of claim 18 wherein said antibody is selected from the polyclonal antibody listeria (Thermo Fischer Scientific number PA1-85650); Samonela polyclonal antibody (Thermo Fischer Scientific, under reference PA1-73022; E. Coli polyclonal antibody (Thermo Fisher Scientific number PA1-73035) or Staphylococcus aureus polyclonal antibody (Thermo Fisher Scientific number PA1-73174).

**35.** The system of claim 18 wherein said coated substrate is selected from a magnetic substrate.

**36.** The system of claim 18 wherein said coated substrate is selected from a non-magnetic substrate.

**37.** The system of claim 18 wherein said coated substrate is coated with a thin film of a noble metal or a material that chemically interacts with thiol groups.

**38.** The system of claim 37 wherein said noble metal is selected from gold.

**39.** The system of claim 37 wherein said material that chemically interacts with thiol groups is selected from silver (Ag), copper (Cu) or zinc oxide (ZnO).

FIGURE 1

**FIGURE 2**

**FIGURE 3A**

**FIGURE 3B**

**FIGURE 4**

**FIGURE 5A**

**FIGURE 5B**

**FIGURE 6**

**FIGURE 7**

**FIGURE 8**

**FIGURE 9A**

**FIGURE 9B**

**FIGURE 9C**

FIGURE 10

FIGURE 11

**FIGURE 12**

# INTERNATIONAL SEARCH REPORT

International application No.
PCT/CL2019/050164

**A. CLASSIFICATION OF SUBJECT MATTER**

CIP: G01N33/543, G01N33/553, G01N27/72 (2020.01)

According to International Patent Classification (IPC) or to both national classification and IPC

**B. FIELDS SEARCHED**

Minimum documentation searched (classification system followed by classification symbols)

CIP: G01N33/543, G01N33/553, G01N27/72        CPC: G01N33/54326, G01N27/745

Documentation searched other than minimum documentation to the extent that such documents are included in the fields searched

Electronic data base consulted during the international search (name of data base and, where practicable, search terms used)

STN, ESPACENET, PATENTSCOPE, GOOGLE SCHOLAR, INAPI

**C. DOCUMENTS CONSIDERED TO BE RELEVANT**

| Category* | Citation of document, with indication, where appropriate, of the relevant passages | Relevant to claim No. |
|---|---|---|
| X --------- Y | LIU, X. et al. Surface plasmon resonance immunosensor for fast, highly sensitive, and *in situ* detection of the magnetic nanoparticles-enriched *Salmonella enteritidis*. Sensors and Actuators B: Chemical, 2016; 230:191-198. DOI: 10.1016/j.snb.2016.02.043. **The whole document** | 1-3, 5-12, 14-16 -------------------------- 4 |
| Y | SHI, L. et al. A novel method to detect *Listeria monocytogenes* via superparamagnetic lateral flow immunoassay. Analytical and bioanalytical chemistry, 2015; 407 (2): 529-535. DOI: 10.1007/s00216-014-8276-8 **The whole document** | 4, 12, 26, 34 |
| X | MANTECA, A. et al. GMR sensors: Magnetoresistive behaviour optimization for biological detection by means of superparamagnetic nanoparticles. Biosensors and Bioelectronics, 2011; 26(8): 3705-3709. DOI:10.1016/j.bios.2011.02.013 **The whole document** | 1, 5, 6,13, 15,16,17 |

| [X] Further documents are listed in the continuation of Box C. | [X] See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30/07/2020        **30/July/2020** | 18/08/2020        **18/August/2020** |

| Name and mailing address of the ISA/ INAPI, Av. Libertador Bernardo O'Higgins 194, Piso 17, Santiago, Chile Facsimile No. | Authorized officer GARRIDO, Carolina Telephone No. |
|---|---|

Form PCT/ISA/210 (second sheet) (January 2015)

## INTERNATIONAL SEARCH REPORT

International application No.

PCT/CL2019/050164

C (Continuation). DOCUMENTS CONSIDERED TO BE RELEVANT

| Category* | Citation of document, with indication, where appropriate, of the relevant passages | Relevant to claim No. |
|---|---|---|
| X<br>--------<br>Y | SUN, X. et al. Separable detecting of *Escherichia coli O157H: H7* by a giant magneto-resistance-based bio-sensing system. Sensors and Actuators B: Chemical, 2016; 234: 485-492. DOI:10.1016/j.snb.2016.04.183.<br>**The whole document** | 1-3, 5-11, 13-25, 27-33, 35-39<br>----------------------<br>4, 12, 26, 34 |
| A | HA, Y. et al. Recent Advances Incorporating Superparamagnetic Nanoparticles into Immunoassays. ACS applied nano materials, 2018; (1,2): 512-521. DOI:10.1021/acsanm.7b00025.<br>**The whole document** | |
| A | KRISHNA, V. D. et al. Nanotechnology: Review of concepts and potential application of sensing platforms in food safety. Food microbiology, 2018; 75: 47-54. DOI: 10.1016/j.fm.2018.01.025.<br>**The whole document** | |
| A | US 2018/0128822 A1 (MAGARRAY, INC.) **10 May 2018**<br>**The whole document** | |
| A | US 5,981,297 A (THE UNITED STATES OF AMERICA AS REPRESENTED BY THE SECRETARY OF THE NAVY) **09 November 1999**<br>**The whole document** | |

Form PCT/ISA/210 (continuation of second sheet) (January 2015)

INTERNATIONAL SEARCH REPORT

Information on patent family members

International application No.

PCT/CL2019/050164

| US 2018/0128822 A1 | 10-05-2018 | CN101868286 (A) | 20-10-2010 |
| | | CN105606799 (A) | 25-05-2016 |
| | | CN105606799 (B) | 19-02-2019 |
| | | EP2200727 (A1) | 30-06-2010 |
| | | EP2200727 (A4) | 15-12-2010 |
| | | EP2200727 (B1) | 13-08-2014 |
| | | EP2728357 (A1) | 07-05-2014 |
| | | EP2728357 (B1) | 22-02-2017 |
| | | US2009104707 (A1) | 23-04-2009 |
| | | US9863939 (B2) | 09-01-2018 |
| | | WO2009039437 (A1) | 26-03-2009 |
| US 5,981,297 A | 09-11-1999 | None | |

Form PCT/ISA/210 (patent family annex) (January 2015)

**EP 4 086 629 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8679458 B2
- WO 2003057175 A3
- US 8815610 B2
- US 8318093 B2
- EP 2385386 A1

**Non-patent literature cited in the description**

- **DR BASELT et al.** *Holidays. Sci. Technol., B,* 1996, vol. 14, 789-794
- **G U LEE et al.** *Bioanalytical Chemistry,* 2000, vol. 287, 261-271
- **DR BASELT et al.** *Biosensor Bioelectronics,* 1998, vol. 13, 731-739
- **X. QU ; F. WANG ; Y. SUN ; Y. TIAN ; R. CHEN ; X. MA ; C. LIU.** Selective extraction of bioactive glycoprotein in neutral environment through Concanavalin A mediated template immobilization and dopamine surface imprinting. *RSC Adv.,* 2016, vol. 6, 86455-86463
- **Y. MAO ; X. HUANG ; S. XIONG ; H. XU ; ZP AGUILAR ; Y. XIONG.** Large-volume immunomagnetic separation combined with multiplex PCR assay for simultaneous detection of Listeria monocytogenes and Listeria ivanovii in lettuce. *Food Control.,* 2016, vol. 59, 601-608, https://doi.org/10.1016/j.foodcont.2015.06.048
- **Y. XUE ; X. LI ; H. LI ; W. ZHANG.** Quantifying thiol - gold interactions towards the efficient strength control. *Nat. Commun.,* 2014, vol. 5, 4348, http://dx.doi.org/10.1038/ncomms5348
- **M. JIANG ; X. PENG.** Anisotropic Fe3O4/Mn3O4 Hybrid Nanocrystals with Unique Magnetic Properties. *Nano Lett.,* 2017, vol. 17, 3570-3575
- **VAN DER PAUW, LJ.** *Philips Research Reports.,* 1958, vol. 13, 1-9
- **VAN DER PAUW, LJ.** *Philips Technical Review,* 1958, vol. 20, 220-224